# EUROPEAN PATENT SPECIFICATION

(11) **EP 2 051 686 B1**
(45) Date of publication and mention of the grant of the patent: **18.05.2011**
(21) Application number: 07825986.8
(22) Date of filing: 03.08.2007
(51) Int. Cl.: A61K 8/02, A61K 8/86, A61K 8/891, A61Q 19/00

(54) **SOFT AND ABSORBENT TISSUE PRODUCTS**
WEICHE, SAUGFÄHIGE TISSUE PRODUKTE
PRODUITS EN TISSU SOUPLE ET ABSORBANT

(30) Priority: 26.10.2006 US 588076
(43) Date of publication of application: 29.04.2009
(73) Proprietor: KIMBERLY-CLARK WORLDWIDE, INC., Neenah, WI 54956 (US)
(72) Inventor: LIU, Kou-Chang, Neenah, WI 54956 (US)
(74) Representative: Beacham, Annabel Rose
(86) International application number: PCT/IB2007/053070
(87) International publication number: WO 2008/050244

(56) References cited:
- WO-A-96/08601
- US-A1- 2005 136 097
- US-A1- 2006 130 989

## Description

### Background of the Invention

The use of various polysiloxanes to soften tissue products, such as facial and bath tissue, is well known in the tissue industry. However, while polysiloxanes are effective in providing a smooth surface feel, they are generally hydrophobic and retard wettability. In addition, polysiloxanes can be expensive. Therefore there is a need for lower cost polysiloxane compositions which provide the desired level of softness without reducing the wettability of the tissue to unacceptable levels.

US2006/130989 describes a softening composition for treating tissue products comprising amino-functional polysiloxanes, alkoxylated alcohols and additives. D1 contains only two components, i.e. 5-30 wt% of an amino-functional polysiloxane and 70-95 wt% of a fatty alkyl derivative.

US2005/136097 discloses a soft paper based product comprising a softening composition comprising polysiloxanes, emollient and water.

WO96/08601 discloses a tissue treating agent comprising a softening composition which comprises a polysiloxane and a polyethylene glycol.

### Summary of the Invention

It has now been discovered that certain blends of components can provide tissues with the desired balance of softness and wettability at a reduced cost compared to current polysiloxane softness compositions. More specifically, it has been found that polysiloxanes can be combined with one or more polyalkylene oxides and/or one or more fatty alkyl derivatives in the proper ratios, particularly when the polyalkylene oxide(s) and/or the fatty alkyl derivative(s) is(are) solid at room temperature, to provide improved softness compared to the polysiloxane alone, as well as good wettability for tissue products, particularly facial tissues.

The invention resides in a tissue sheet having a topically-applied softening composition which comprises, on a solids basis, from 30 to 75 weight percent of one or more polysiloxanes, from 1 to 60 weight percent of one or more polyalkylene oxides and from 1 to 60 weight percent of one or more fatty alkyl derivatives, wherein the combined amount of the polyalkylene oxide(s) and the fatty alkyl derivative(s) is about 25 weight percent or greater.

The softening composition can topically appied onto one or both of the outer tissue product surfaces, such as by printing or spraying, or by any other manner known in the tissue making art. Topical addition tends to concentrate the softening composition on the surface(s) of the tissue product where its softening characteristics are most readily apparent to the consumer. The add-on amount can be from 0.5 to 10 weight percent, more specifically from 0.5 to 5 weight percent, and still more specifically from 1 to 3 weight percent.

Polysiloxanes useful for purposes of this invention can have one or more pendant functional groups such as amine, quaternium, aldehyde, epoxy, hydroxy, alkoxyl, polyether and carboxylic acid and its derivatives, such as amides and esters. Suitable polysiloxanes can have the following general structure: 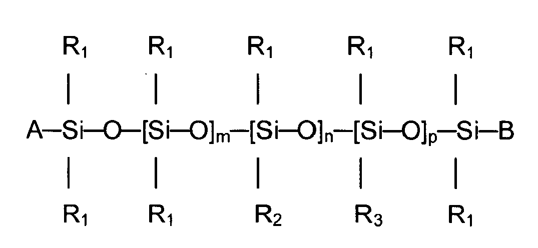 wherein:
"m" is from10 to100,000;
"n" is from 1 to 10,000;
"p" is from 0 to 1,000;
"A" and "B" are independently a hydroxyl, C₁ to C₂₀ or R₂;
R₁, R₂ and R₃ are distributed in random or block fashion;
R₁ is a C₁ to C₈ radical, which can be straight chain, branched or cyclic;
R₂ is a C₁ to C₈ radical, which can be straight chain, branched or cyclic, or of the structure: 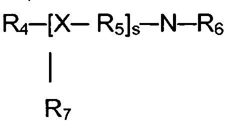 wherein
   R₄ and R₅ are independently a C₂ to C₈ alkylene diradical, which can be straight chain or branched, substituted, or unsubstituted;
   X is an oxygen or N-R₈;
   R₆, R₇ and R₈ are independently hydrogen, a substituted or unsubstituted C₁ or C₂, a substituted or unsubstituted straight chain or branched or cyclic C₃ to C₂₀ alky radical, or an acyl radical, such as an acetyl radical; and
   "s" is 0 or1;
R₃ is of the structure: R₉-Y-[C₂H₄O]ᵣ-[C₃H₆O]_{q}-R₁₀
   wherein
   Y is an oxygen or N-R₁₁;
   R₉ is a C₂ to C₈ alkylene diradical, which can be straight chain or branched, substituted or unsubstituted;
   R₁₀ and R₁₁ are independently hydrogen, a substituted or unsubstituted C₁ or C₂, a substituted or unsubstituted, straight chain or branched or cyclic C₃ to C₂₀ alkyl radical;
   "r" is from 1 to 100,000; and
   "q" is from 0 to 100,000.
When R₂ = R₁, "A" and "B" can also be a nitrogen quarternium.

Examples of suitable commercially available polysiloxanes include AF-2340, AF-2130, HAF-1130, EAF-3000, EAF-340, EAF-15, AF-2740, WR-1100, WR-1300 and Wetsoft CTW from Kelmar/Wacker; DC-8822, DC-8566, DC-8211, DC-SF8417, DC-2-8630, DC-NSF, DC-8413, DC-SSF, DC-8166 from Dow Corning; SF-69, SF-99 SF-1023 from GE Silicones and Tegopren 6924, Tegopren 7990,Tego IS4111 from Goldschmidt/Degussa.

The amount of the polysiloxane in the softening composition, on a solids basis, can be from 30 to 75 weight percent, more specifically from 30 to 70 weight percent, more specifically from 40 to 70 weight percent, and still more specifically from 50 to 70 weight percent.

Polyalkylene oxides suitable for purposes of this invention can have the following general structure:

R₁₂-[C₂H₄O}ᵢ-[C₃H₆O]ⱼ-[CₜH₂ₜO]ᵥ-R₁₃

wherein:
R₁₂ and R₁₃ are independently a hydrogen, a substituted or unsubstituted C₁ to C₆ alkyl radical, a straight chain or branched C₁ to C₆ alkyl radical, or a cyclic C₁ to C₆ alkyl radical;
"i", "j" and "v" are independently from 0 to 100,000, with the oxide moieties are distributed along the polymer backbone randomly or as blocks;
"i + j + v" is equal to or greater than 10; and
"t" is from 4 to 10.

The polyalkylene oxide can be in a liquid or solid state at room temperature. However, a polyakylene oxide which is solid at room temperature is preferred. Examples of suitable commercially available polyalkylene oxides are Carbowax PEG 600, Carbowax PEG 1450 and Carbowax PEG 8000 from Dow Chemical.

The amount of the polyalkylene oxide in the softening composition, on a solids basis, is from 1 to 60 weight percent, more specifically from 10 to 60 weight percent, more specifically from 20 to 60 weight percent, more specifically from 30 to 60 weight percent, and still more specifically from 30 to 50 weight percent.

Fatty alkyl derivatives suitable for purposes of this invention can have the following general structure:

R₁₄-G

wherein:
R₁₄ is a C₈ to C₄₀ alkyl radical, which can be substituted or unsubstituted, primary, secondary or tertiary; straight chain, branched or cyclic; and
"G" is hydroxy, amine, sulfonate, sulfate, phosphate, acid or acid derivative, or -Q-[C₂H₄O]ᵢ-[C₃H₆O]ⱼ-[CₜH₂ₜO]ᵥ-R₁₃ radical;
   wherein
   "Q" is an oxygen radical, an NH radical or N-[C₂H₄O}ᵢ-C₃H₆O]ⱼ-[CₜH₂ₜO]ᵥ-R₁₃ radical;
   R₁₃ is a hydrogen, a substituted or unsubstituted C₁ to C₆ alkyl radical, a straight chain or branched C₁ to C₆ alkyl radical, or a cyclic C₁ to C₆ alkyl radical;
   "i", "j" and "v" are independently from 0 to 100,000, where the oxide moieties are distributed along the polymer backbone randomly or as blocks;
   "i + j + v" is equal to or greater than 10; and
   "t" is from 4 to 10.

The fatty alkyl derivatives can be in liquid or solid state at room temperature. However, a fatty alkyl derivative which is a solid at room temperature is preferred. Examples of commercially available suitable fatty alkyl derivatives are glycerol stearate, glycerol dilaurate, sorbitan monopalmitate, sorbitan tristearate, sorbitan sesquioleate polyoxyethylene sorbitan palmitate, 9-EO ethoxylated tridecylalcohol, Ceteth-10, Ceteth-12 (12-EO ethoxylated cetyl alcohol) and Ceteth-20. More particularly, suitable commercially available fatty alkyl derivatives include Pluraface A-38, Macol CSA 20 and Macol LA 12 from BASF; Armeen 16D, Armeen 18D, Armeen HTD, Armeen 2C, Armeen M2HT, Armeen 380, Ethomeen 18/15 Armid O, Witconate 90, Witconate AOK, and Witcolate C from Akzo Nobel.

The amount of the fatty alkyl derivative in the softening composition, on a solids basis, is 1 to 60 weight percent, more specifically from 1 to 50 weight percent, more specifically from 10 to 50 weight percent, more specifically from 20 to 50 weight percent, and still more specifically from 20 to 40 weight percent.

In the interests of brevity and conciseness, any ranges of values set forth in this specification are to be construed as written description support for claims reciting any sub-ranges having endpoints which are whole number values within the specified range in question. By way of a hypothetical illustrative example, a disclosure in this specification of a range of from 1 to 5 shall be considered to support claims to any of the following sub-ranges: 1-4; 1-3; 1-2; 2-5; 2-4; 2-3; 3-5; 3-4; and 4-5.

### Examples

Three-ply, wet-pressed, creped facial tissue products were made with different softening compositions of this invention as described below and tested for softness and wettability. In general, the tissue base sheets were produced using a conventional wet-pressed tissue making process well known in the art. More particularly, an aqueous suspension of papermaking fibers was issued from a layered headbox onto a forming fabric. The furnish consisted of 70 weight percent hardwood (eucalyptus) fibers and 30 weight percent softwood fibers. A vacuum box beneath forming fabric was adapted to remove water from the fiber furnish to assist in forming a web. The newly formed web was transferred to a felt with aid of a pick up roll. While supported by the felt, the tissue web was lightly pressed onto the surface of a Yankee dryer using a press roll. The dried web was creped from the surface of the Yankee dryer and the resulting single-ply tissue base sheet was wound onto a parent roll. Thereafter, the base sheets from three like parent rolls were unwound and converted into a three-ply basesheet for subsequent application of the various softening compositions. The finished basis weight of the three-ply base sheet was about 22.7 pounds per 2880 square feet.

The softening composition was simultaneously appied to both surfaces of the three-ply basesheet by rotogravure printing. The gravure rolls were electronically engraved, chrome over copper rolls supplied by Southern Graphics Systems, located at Louisville, Ky. The rolls had a line screen of 360 cells per lineal inch and a volume of 1.5 Billion Cubic Microns (BCM) per square inch of roll surface. Typical cell dimensions for this roll were 65 microns in length, 110 microns in width, and 13 microns in depth. The rubber backing offset applicator rolls were a 75 Shore A durometer cast polyurethane supplied by American Roller Company, located at Union Grove, Wisconsin. The process was set up to a condition having 0.375 inch interference between the gravure rolls and the rubber backing rolls and 0.003 inch clearance between the facing rubber backing rolls. The simultaneous offset/offset gravure printer was run at a speed of 2000 feet per minute. This process yielded a solids add-on level of about 1.0 weight percent based on the dry weight of the finished tissue product. (0.5 dry weight percent on each side of the product.

After the tissue products were made, they were tested for geometric mean tensile strength, wettability and softness.

As used herein, the "geometric mean tensile strength" (GMT) is the square root of the product of the dry machine direction tensile strength multiplied by the dry cross-machine direction tensile strength and is expressed as grams per 3 inches of sample width. The machine direction tensile strength is the peak load per 3 inches of sample width when a sample is pulled to rupture in the machine direction. Similarly, the cross-machine direction (CD) tensile strength is the peak load per 3 inches of sample width when a sample is pulled to rupture in the cross-machine direction. More specifically, samples for tensile strength testing are prepared by cutting a 3 inches (76.2 mm) wide by 5 inches (127 mm) long strip in either the machine direction (MD) or cross-machine direction (CD) orientation using a JDC Precision Sample Cutter (Thwing-Albert Instrument Company, Philadelphia, PA, Model No. JDC 3-10, Serial No. 37333). The instrument used for measuring tensile strengths is an MTS Systems Sintech 11S, Serial No. 6233. The data acquisition software is MTS TestWorks® for Windows Ver. 3.10 (MTS Systems Corp., Research Triangle Park, NC). The load cell is selected from either a 50 Newton or 100 Newton maximum, depending on the strength of the sample being tested, such that the majority of peak load values fall between10-90% of the load cell's full scale value. The gauge length between jaws is 4 +/-0.04 inches (101.6 +/-1 mm). The jaws are operated using pneumatic-action and are rubber coated. The minimum grip face width is 3 inches (76.2 mm), and the approximate height of a jaw is 0.5 inches (12.7 mm). The crosshead speed is 10 +/- 0.4 inches/min (254 +/-1 mm/min), and the break sensitivity is set at 65%. The sample is placed in the jaws of the instrument, centered both vertically and horizontally. The test is then started and ends when the specimen breaks. The peak load is recorded as either the "MD tensile strength" or the "CD tensile strength" of the specimen depending on direction of the sample being tested. At least six (6) representative specimens are tested for each product or sheet, taken "as is", and the arithmetic average of all individual specimen tests is either the MD or CD tensile strength for the product or sheet.

Wettability of the tissue products is determined by the "Wet Out Time", which is related to absorbency, and is the time it takes for a prepared sample to completely wet out when placed in water. More specifically, the Wet Out Time is determined by cutting 20 product sheets of the tissue sample into 2.5 inch squares. The number of product sheets used in the test is independent of the number of plies per sheet of product. (For a 3-ply product were are 60 plies in each pad.) The 20 square sheets are stacked together and stapled at each corner to form a pad. The pad is held close to the surface of a constant temperature distilled water bath (23 ± 2 °C), which is the appropriate size and depth to ensure the saturated specimen dose not cntact the bottom of the container and the top surface of the water at the same time, and dropped flat onto the water surface, staple points down. The time taken for the pad to become completely saturated, measured in seconds, is the Wet out Time for the sample and represents the absorpbent rate of the tissue. Increases in the Wet Out Time represent a decrease in the absorbent rate.

Softness of the tissue products was determined by a trained in-hand ranking panel, which provides a basic assessment of the softness and stiffness characteristics of a tissue product. The ranking panel is trained to provide holistic assessments as close as possible to those that a typical consumer might provide. In carrying out the test, two different assessments are made: Softness and Softness-on-Face. The Softness test involves evaluating the velvety, silky or fuzzy feel of the tissue sample when rubbed between the thumb and fingers. The Softness-on-Face test involves rubbing the tissue sample against the face, including the area between the nose and lips. Rank data generated for each sample code by the panel are analyzed using a proportional hazards regression model. This model assumes computationally that the panelist proceeds through the ranking procedure from most of the attribute being assessed to least of the attribute. The softness test results are presented in the tables below as log odds values. The log odds are the natural logarithm of the risk ratios that are estimated for each code from the proportional hazards regression model. Larger log odds indicate the attribute of interest is perceived with greater intensity.

### Example 1.

Two tissue softening compositions were prepared and supplied by the Kelmar Division of Wacker Chemical Corporation, Duncan, SC. The relevant chemical components of the formulations are set forth in Table 1. Tissue samples were prepared as described above by gravure coating the two formulations on a three-ply, wet-pressed, creped facial tissue basesheet. A total add-on of 1 weight percent (0.5 weight percent on each side) was evenly coated on both sides of the basesheet. The treated basesheets were then converted into folded facial tissue products. (The percentage of each component, on a solids basis, is in parentheses).

**TABLE 1**

| Formulation | Polysiloxane AF-23 | 12-EO Ethoxylated Cetyl alcohol | 9-EO Ethoxylated tridecyl alcohol | Water and other formulation aids balance to 100% |
|---|---|---|---|---|
| 1 (Control) | 30% (83%) | 0% (0%) | 6% (17%) | " |
| 2 (Invention) | 22.5% (67%) | 5% (15%) | 6% (18%) | " |

The GMT and Wet Out Time of the facial tissue products were measured and the Softness and Softness-on-Face of the tissue products were evaluated. The results are set forth in Table 2 below.

**TABLE 2**

| Formulation | GMT | WOT | Soft on Face | Softness |
|---|---|---|---|---|
| 1 (Control) | 933 | 34.5 | 0.2308 | -0.0646 |
| 2 (Invention) | 929 | 25.6 | 0.9738 | 0.3842 |

The results illustrate that at similar physical strength (GMT), the tissue product treated with the softening chemical composition of this invention (Formulation 2) has a shorter Wet Out Time (or better wettability) and was softer in general and softer on the face when compared with the tissue product treated with the control (Formulation 1) (In Formulation 1, the combined amount of the polyalkylene oxide(s) and the fatty alkyl derivative(s) is less than 25 weight percent.)

### Example 2.

Softening compositions designated as Formulations 3 - 8 in Table 3 were prepared, applied to tissue samples and tested as described in Example 1. (The percentage of each component, on a solids basis, is in parentheses). The test results are set forth in Table 4.

**TABLE 3**

| Formulation | Polysiloxane (AF-23) | Polyethylene oxide (PEG 1450) | 9-EO Ethoxylated tridecylalcohol | Water and other formulation aids balance to 100% |
|---|---|---|---|---|
| 3 (Control) | 30% (83%) | 0% (0%) | 6% (17%) | " |
| 4 (Invention) | 21% (58%) | 9% (25%) | 6% (17%) | " |
| 5 (Invention) | 18% (50) | 12% (33%) | 6% (17%) | " |
| 6 (Invention) | 15% (42%) | 15% (42%) | 6% (17%) | " |
| 7 (Invention) | 12% (33%) | 18% (50%) | 6% (17%) | " |
| 8 (Invention) | 9% (25%) | 21% (58%) | 6% (17%) | " |

**TABLE 4**

| Formulation | GMT | WOT | Soft-on-Face | Softness |
|---|---|---|---|---|
| 3 (Control) | 964 | 32.2 | 0.5053 | -0.0344 |
| 4 (Invention) | 975 | 25.6 | 0.7572 | 0.1405 |
| 5 (Invention) | 980 | 30.3 | 0.9222 | -0.1781 |
| 6 (Invention) | 979 | 30.9 | 0.8172 | 0.1056 |
| 7 (Invention) | 971 | 22.9 | 0 | 0 |
| 8 (Invention) | 1043 | 16.7 | 0.3191 | -1.0158 |

The results shown in Table 4 illustrate that at similar physical strength (GMT), tissue products treated with the softening compositions of this invention, such as the tissue product treated with Formulation 4, has a shorter Wet Out Time (better wettability) and was softer in general and softer on the face when compared with the tissue product treated with the control (Formulation 3).

It will be appreciated that the foregoing description and examples, given for purposes of illustration, are not to be construed as limiting the scope of this invention, which is defined by the following claims and all equivalents thereto.

## Claims

1. A tissue sheet having from 0.5 to 10 dry weight percent of a topically-applied softening composition which comprises, on a solids basis, from 30 to 75 weight percent of one or more polysiloxanes, from 1 to 60 weight percent of one or more polyalkylene oxides and from 1 to 60 weight percent of one or more fatty alkyl derivatives, wherein the combined amount of the polyalkylene oxide(s) and the fatty alkyl derivative(s) is about 25 weight percent or greater.

2. The tissue sheet of claim 1 wherein one or more of the polysiloxanes has the following general structure: 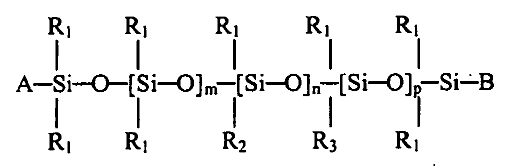 wherein:
"m" is from 10 to 100,000;
"n" is from 1 to 10,000;
"p" is from 0 to 1,000;
"A" and "B" are independently a hydroxyl, C₁ to C₂₀ or R₂;
R₁, R₂ and R₃ are distributed in random or block fashion;
R₁ is a C₁ to C₈ radical, which can be straight chain, branched or cyclic;
R₂ is a C₁ to C₈ radical, which can be straight chain, branched or cyclic, or of the structure: 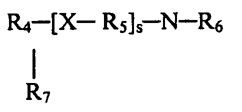 wherein
R₄ and R₅ are independently a C₂ to C₈ alkylene diradical, which can be straight chain or branched, substituted, or unsubstituted;
X is an oxygen or N-R₈;
R₆, R₇ and R₈ are independently hydrogen, a substituted or unsubstituted C₁ or C₂, a substituted or unsubstituted straight chain or branched or cyclic C₃ to C₂₀ alky radical, or an acyl radical, such as an acetyl radical; and "s" is 0 or 1;
R₃ is of the structure: R₉-Y-[C₂H₄O]ᵣ-[C₃H₆O]_{q}-R₁₀
wherein
Y is an oxygen or N-R₁₁;
R₉ is a C₂ to C₈ alkylene diradical, which can be straight chain or branched, substituted or unsubstituted;
R₁₀ and R₁₁ are independently hydrogen, a substituted or unsubstituted C₁ or C₂, a substituted or unsubstituted, straight chain or branched or cyclic C₃ to C₂₀ alkyl radical;
"r" is from 1 to 100,000; and
"q" is from 0 to 100,000.

3. The tissue of claim 2 wherein R₂ is an alkylene substituted with a diamine or a mono-amine.

4. The tissue of claim 2 wherein R₂ = R₁ and "A" and "B" are a nitrogen quartemium.

5. The tissue of claim 1 wherein the polyalkylene oxide has the following general structure:
R₁₂-[C₂H₄O]ᵢ-[C₃H₆O]ⱼ-[CₜH₂ₜO]ᵥ-R₁₃
wherein:
R₁₂ and R₁₃ are independently a hydrogen, a substituted or unsubstituted C₁ to C₆ alkyl radical, a straight chain or branched C₁ to C₆ alkyl radical, or a cyclic C₁ to C₆ alkyl radical;
"i", "j" and "v" are independently from 0 to 100,000, with the oxide moieties are distributed along the polymer backbone randomly or as blocks;
"i + j + v" is equal to or greater than 10; and
"t" is from 4 to 10.

6. The tissue of claim 4 wherein the polyakylene oxide is a polyethylene oxide or a polyethylene glycol, preferably with a molecular weight greater than 600.

7. The tissue of claim 1 wherein the polyalkylene oxide is a solid at room temperature.

8. The tissue of claim 1 wherein the fatty alkyl derivative has the following general structure:
R₁₄-G
wherein:
R₁₄ is a C₈ to C₄₀ alkyl radical, which can be substituted or unsubstituted, primary, secondary or tertiary; straight chain, branched or cyclic; and
"G" is hydroxy, amine, sulfonate, sulfate, phosphate, acid or acid derivative, or -Q-[C₂H₄O]ᵢ-[C₃H₆O]ⱼ-[CₜH₂ₜO]ᵥ-R₁₃ radical;
wherein
"Q" is an oxygen radical, an NH radical or N-[C₂H₄O}ᵢ-[C₃H₆O]ⱼ-[CₜH₂ₜO]ᵥ-R₁₃ radical;
R₁₃ is a hydrogen, a substituted or unsubstituted C₁ to C₆ alkyl radical, a straight chain or branched C₁ to C₆ alkyl radical, or a cyclic C₁ to C₆ alkyl radical;
"i", "j" and "v" are independently from 0 to 100,000, where the oxide moieties are distributed along the polymer backbone randomly or as blocks;
"i + j + v" is equal to or greater than 10; and
"t" is from 4 to 10.

9. The tissue of claim 1 wherein the fatty alkyl derivative is an ethoxylated alcohol or a mixtures of ethoxylated alcohols, and preferably has a carbon chain length of 12 or greater, or is an ethoxylated tridecyl alcohol or ethoxylated cetyl alcohol.

10. The tissue of claim 8 wherein the fatty alkyl derivative is a solid at room temperature.

11. The tissue of claim 1 wherein the polysiloxane is an amino-derivatized polysiloxane and the fatty alkyl derivative is an alkylenoxylated alcohol, or an alkylenoxylated amine.

12. The tissue of claim 1 wherein the tissue softening composition comprises about 70 weight of an amino-polysiloxane and about 30 weight percent of an ethoxylated alcohol or a mixture of ethoxylated alcohols, and preferably wherein the ethoxylated alcohols are in solid form at room temperature.

13. The tissue of claim 1 wherein the tissue softening composition comprises from 50 to 70 weight percent of an amino-polysiloxane and from 30 to 50 weight percent polyethylene glycol, and preferably wherein the polyethylene glycol is a solid at room temperature.

14. The tissue of claim 1 wherein the tissue softening composition comprise from 50 to 70 weight percent of an amino-polysiloxane and from 30 to 50 weight percent of a mixture of at least one polyethylene glycol and at least one fatty alkyl derivative, and preferably wherein the polyethylene glycol and fatty alkyl derivative are in solid form at room temperature.

15. The tissue of claim 1 wherein the fatty alkyl derivative is glycerol stearate, glycerol dilaurate, sorbitan monopalmitate, sorbitan tristearate, sorbitan sesquioleate, polyoxyethylene sorbitan palmitate, 9-EO ethoxylated tridecylalcohol, ceteth-10, ceteth-12 or ceteth-20.

## Patentansprüche

1. Tissueblatt mit 0,5 bis 10 Trockengewichtsprozent einer topisch applizierten Weichmacherzusammensetzung, die auf Feststoffbasis 30 bis 75 Gewichtsprozent eines oder mehrerer Polysiloxane, 1 bis 60 Gewichtsprozent eines oder mehrerer Polyalkylenoxide und 1 bis 60 Gewichtsprozent eines oder mehrerer Fettalkylderivate umfaßt, wobei die kombinierte Menge des Polyalkylenoxids bzw. der Polyalkylenoxide und des Fettalkylderivats bzw. der Fettalkylderivate etwa 25 Gewichtsprozent oder mehr beträgt.

2. Tissueblatt nach Anspruch 1, wobei eines oder mehrere der Polysiloxane die folgende allgemeine Struktur aufweist bzw. aufweisen: 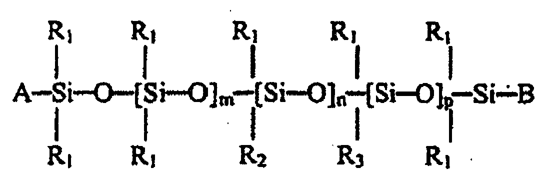 worin:
"m" für 10 bis 100.000 steht;
"n" für 1 bis 10.000 steht;
"p" für 0 bis 1000 steht;
"A" und "B" unabhängig voneinander für Hydroxyl, C₁ bis C₂₀ oder R₂ stehen;
R₁, R₂ und R₃ statistisch oder blockartig verteilt sind;
R₁ für einen C₁-C₈-Rest, der geradkettig, verzweigt oder cyclisch sein kann, steht;
R₂ für einen C₁-C₈-Rest, der geradkettig, verzweigt oder cyclisch sein kann, oder mit der Struktur: 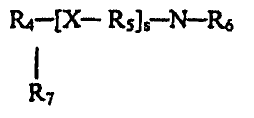 worin
R₄ und R₅ unabhängig voneinander für einen zweiwertigen C₂-C₈-Alkylenrest, der geradkettig oder verzweigt und substituiert oder unsubstituiert sein kann, stehen;
X für Sauerstoff oder N-R₈ steht;
R₆, R₇ und R₈ unabhängig voneinander für Wasserstoff, substituiertes oder unsubstituiertes C₁ oder C₂, einen substituierten oder unsubstituierten geradkettigen oder verzweigten oder cyclischen C₃- bis C₂₀-Alkylrest oder einen Acylrest, wie einen Acetylrest, stehen und "s" für 0 oder 1 steht;
steht;
R₃ die Struktur: R₉-Y-[C₂H₄O]ᵣ-[C₃H₆O]_{q}-R₁₀ aufweist,
worin
Y für Sauerstoff oder N-R₁₁ steht;
R₉ für einen zweiwertigen C₂-C₈-Alkylenrest, der geradkettig oder verzweigt und substituiert oder unsubstituiert sein kann, steht;
R₁₀ und R₁₁ unabhängig voneinander für Wasserstoff, substituiertes oder unsubstituiertes C₁ oder C₂, einen substituierten oder unsubstituierten geradkettigen oder verzweigten oder cyclischen C₃- bis C₂₀-Alkylrest stehen;
"r" für 1 bis 100.000 steht und
"q" für 0 bis 100.000 steht.

3. Tissue nach Anspruch 2, wobei R₂ für ein durch ein Diamin oder ein Monoamin substituiertes Alkylen steht.

4. Tissue nach Anspruch 2, wobei R₂ = R₁ und "A" und "B" für ein Stickstoff-Quarternium stehen.

5. Tissue nach Anspruch 1, wobei das Polyalkylenoxid die folgende allgemeine Struktur aufweist:
R₁₂-[C₂H₄O]ᵢ-[C₃H₆O]ⱼ- [CₜH₂ₜO]ᵥ-R₁₃
worin:
R₁₂ und R₁₃ unabhängig voneinander für Wasserstoff, einen substituierten oder unsubstituierten C₁- bis C₆-Alkylrest, einen geradkettigen oder verzweigten C₁- bis C₆-Alkylrest oder einen cyclischen C₁- bis C₆-Alkylrest stehen;
"i", "j" und "v" unabhängig voneinander für 0 bis 100.000 stehen, wobei die Oxidgruppierungen statistisch oder blockartig entlang der Polymerhauptkette verteilt sind;
"i + j + v" größer gleich 10 ist und
"t" für 4 bis 10 steht.

6. Tissue nach Anspruch 4, wobei es sich bei dem Polyalkylenoxid um ein Polyethylenoxid oder ein Polyethylenglykol, vorzugsweise mit einem Molekulargewicht von mehr als 600, handelt.

7. Tissue nach Anspruch 1, wobei das Polyalkylenoxid bei Raumtemperatur fest ist.

8. Tissue nach Anspruch 1, wobei das Fettalkylderivat die folgende allgemeine Struktur aufweist:
R₁₄-G
worin:
R₁₄ für einen C₈-C₄₀-Alkylrest, der substituiert oder unsubstituiert, primär, sekundär oder
tertiär, geradkettig, verzweigt oder cyclisch sein kann, steht und
"G" für Hydroxy, Amin, Sulfonat, Sulfat, Phosphat, Säure oder Säurederivat oder einen -Q-[C₂H₄O]ᵢ-[C₃H₆O]ⱼ-[CₜH₂ₜO]ᵥ-R₁₃-Rest,
worin
"Q" für einen Sauerstoffrest, einen NH-Rest oder einen -N- [C₂H₄O]ᵢ-[C₃H₆O]ⱼ-[CₜH₂ₜO]ᵥ-R₁₃-Rest steht;
R₁₃ für Wasserstoff, einen substituierten oder unsubstituierten C₁- bis C₆-Alkylrest, einen geradkettigen oder verzweigten C₁- bis C₆-Alkylrest oder einen cyclischen C₁- bis C₆-Alkylrest stehen;
"i", "j" und "v" unabhängig voneinander für 0 bis 100.000 stehen, wobei die Oxidgruppierungen statistisch oder blockartig entlang der Polymerhauptkette verteilt sind;
"i + j + v" größer gleich 10 ist und
"t" für 4 bis 10 steht;
steht.

9. Tissue nach Anspruch 1, wobei das Fettalkylderivat ein ethoxylierter Alkohol oder eine Mischung von ethoxylierten Alkoholen ist und vorzugsweise eine Kohlenstoffkettenlänge von 12 oder mehr aufweist oder ein ethoxylierter Tridecylalkohol oder ethoxylierter Cetylalkohol ist.

10. Tissue nach Anspruch 8, wobei das Fettalkylderivat bei Raumtemperatur fest ist.

11. Tissue nach Anspruch 1, wobei es sich bei dem Polysiloxan um ein aminoderivatisiertes Polysiloxan handelt und es sich bei dem Fettalkylderivat um einen alkylenoxylierten Alkohol oder ein alkylenoxyliertes Amin handelt.

12. Tissue nach Anspruch 1, wobei die Tissue-Weichmacherzusammensetzung etwa 70 Gewichtsprozent eines Aminopolysiloxans und etwa 30 Gewichtsprozent eines ethoxylierten Alkohols oder einer Mischung von ethoxylierten Alkoholen umfaßt und die ethoxylierten Alkohole vorzugsweise bei Raumtemperatur in fester Form vorliegen.

13. Tissue nach Anspruch 1, wobei die Tissue-Weichmacherzusammensetzung 50 bis 70 Gewichtsprozent eines Aminopolysiloxans und 30 bis 50 Gewichtsprozent Polyethylenglykol umfaßt und das Polyethylenglykol vorzugsweise bei Raumtemperatur in fester Form vorliegt.

14. Tissue nach Anspruch 1, wobei die Tissue-Weichmacherzusammensetzung 50 bis 70 Gewichtsprozent eines Aminopolysiloxans und 30 bis 50 Gewichtsprozent einer Mischung von mindestens einem Polyethylenglykol und mindestens einem Fettalkylderivat umfaßt und das Polyethylenglykol und das Fettalkylderivat vorzugsweise bei Raumtemperatur in fester Form vorliegen.

15. Tissue nach Anspruch 1, wobei es sich bei dem Fettalkylderivat um Glycerinstearat, Glycerindilaurat, Sorbitanmonopalmitat, Sorbitantristearat, Sorbitansesquioleat, Polyoxyethylensorbitanpalmitat, ethoxylierten Tridecylalkohol mit 9 EO, Ceteth-10, Ceteth-12 oder Ceteth-20 handelt.

## Revendications

1. Feuille de papier mousseline comportant de 0,5 à 10 pour cent en poids sec d'une composition assouplissante appliquée localement qui comprend, sur la base des matières solides, de 30 à 75 pour cent en poids d'un ou plusieurs polysiloxanes, de 1 à 60 pour cent en poids d'un ou plusieurs poly(oxydes d'alkylène) et de 1 à 60 pour cent en poids d'un ou plusieurs dérivés d'alkyle gras, dans laquelle la quantité combinée du ou des poly(oxyde(s) d'alkylène) et du ou des dérivé(s) d'alkyle gras est d'environ 25 pour cent en poids ou plus.

2. Feuille de papier mousseline selon la revendication 1 dans laquelle un ou plusieurs des polysiloxanes a la structure générale suivante : 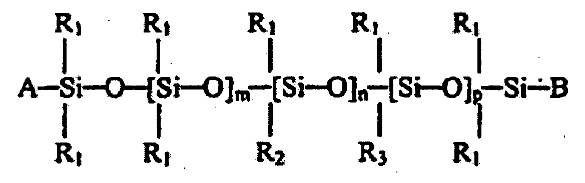 dans laquelle :
"m" a une valeur de 10 à 100 000 ;
"n" a une valeur de 1 à 10 000 ;
"p" a une valeur de 0 à 1 000 ;
"A" et "B" représentent indépendamment un hydroxyle, un groupe en C₁ à C₂₀ ou R₂ ;
R₁, R₂ et R₃ sont distribués de façon statistique ou séquencée ;
R₁ représente un radical en C₁ à C₈, qui peut être à chaîne droite, ramifié ou cyclique ;
R₂ représente un radical en C₁ à C₈, qui peut être à chaîne droite, ramifié ou cyclique, ou de structure : 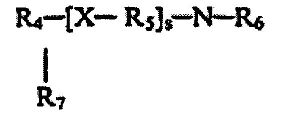 dans laquelle
R₄ et R₅ représentent indépendamment un diradical alkylène en C₂ à C₈, qui peut être à chaîne droite ou ramifié, substitué ou non substitué ;
X représente un oxygène ou N-R₈ ;
R₆, R₇ et R₈ représentent indépendamment un hydrogène, un groupe en C₁ ou C₂ substitué ou non substitué, un radical alkyle en C₃ à C₂₀ à chaîne droite, ou ramifié, ou cyclique, substitué ou non substitué, ou un radical acyle, tel qu'un radical acétyle ; et
"s" est égal à 0 ou 1 ;
R₃ a la structure : R₉-Y-[C₂H4O]ᵣ-[C₃H₆O]_{q}-R₁₀ dans laquelle
Y représente un oxygène ou N-R₁₁ ;
R₉ représente un diradical alkylène en C₂ à C₈, qui peut être à chaîne droite ou ramifié, substitué ou non substitué ;
R₁₀ et R₁₁ représentent indépendamment un hydrogène, un groupe en C₁ ou C₂ substitué ou non substitué, un radical alkyle en C₃ à C₂₀ à chaîne droite, ou ramifié, ou cyclique, substitué ou non substitué ;
"r" a une valeur de 1 à 100 000 ; et
"q" a une valeur de 0 à 100 000.

3. Papier mousseline selon la revendication 2 dans lequel R₂ représente un alkylène substitué par une diamine ou une monoamine.

4. Papier mousseline selon la revendication 2 dans lequel R₂ = R₁ et "A" et "B" représentent un azote quaternaire.

5. Papier mousseline selon la revendication 1 dans lequel le poly(oxyde d'alkylène) a la structure générale suivante :
R₁₂-[C₂H₄O]ᵢ-[C₃H₆O]ⱼ-[CₜH₂ₜO]ᵥ-R₁₃
dans laquelle :
R₁₂ et R₁₃ représentent indépendamment un hydrogène, un radical alkyle en C₁ à C₆ substitué ou non substitué, un radical alkyle en C₁ à C₆ à chaîne droite ou ramifié, ou un radical alkyle en C₁ à C₆ cyclique ;
"i", "j" et "v" ont indépendamment une valeur de 0 à 100 000, les groupements oxyde étant distribués le long de la chaîne principale du polymère de façon statistique ou séquencée ;
"i + j + v" est supérieur ou égal à 10 ; et
"t" a une valeur de 4 à 10.

6. Papier mousseline selon la revendication 4 dans lequel le poly(oxyde d'alkylène) est un poly(oxyde d'éthylène) ou un polyéthylèneglycol, ayant de préférence une masse moléculaire supérieure à 600.

7. Papier mousseline selon la revendication 1 dans lequel le poly(oxyde d'alkylène) est un solide à température ambiante.

8. Papier mousseline selon la revendication 1 dans lequel le dérivé d'alkyle gras a la structure générale suivante :
R₁₄-G
dans laquelle :
R₁₄ représente un radical alkyle en C₈ à C₄₀, qui peut être à chaîne droite, ramifié ou cyclique, primaire, secondaire ou tertiaire, substitué ou non substitué ; et
"G" représente un groupe hydroxy, amine, sulfonate, sulfate, phosphate, acide ou dérivé d'acide, ou un radical -Q-[C₂H₄O]ᵢ-[C₃H₆O]ⱼ-[CₜH₂ₜO]ᵥ-R₁₃ ;
dans lequel
"Q" représente un radical oxygène, un radical NH ou un radical N-[C₂H4O]ᵢ-[C₃H₆O]ⱼ-[CₜH₂ₜO]ᵥ-R₁₃ ;
R₁₃ représente un hydrogène, un radical alkyle en C₁ à C₆ substitué ou non substitué, un radical alkyle en C₁ à C₆ à chaîne droite ou ramifié, ou un radical alkyle en C₁ à C₆ cyclique ;
"i", "j" et "v" ont indépendamment une valeur de 0 à 100 000, les groupements oxyde étant distribués le long de la chaîne principale du polymère de façon statistique ou séquencée ;
"i + j + v" est supérieur ou égal à 10 ; et
"t" a une valeur de 4 à 10.

9. Papier mousseline selon la revendication 1 dans lequel le dérivé d'alkyle gras est un alcool éthoxylé ou un mélange d'alcools éthoxylés, et a de préférence une longueur de chaîne carbonée de 12 ou plus, ou est un alcool tridécylique éthoxylé ou un alcool cétylique éthoxylé.

10. Papier mousseline selon la revendication 8 dans lequel le dérivé d'alkyle gras est un solide à température ambiante.

11. Papier mousseline selon la revendication 1 dans lequel le polysiloxane est un polysiloxane aminé et le dérivé d'alkyle gras est un alkylèneoxy-alcool ou une alkylèneoxy-amine.

12. Papier mousseline selon la revendication 1 dans lequel la composition assouplissante pour papier mousseline comprend environ 70 pour cent en poids d'un amino-polysiloxane et environ 30 pour cent en poids d'un alcool éthoxylé ou d'un mélange d'alcools éthoxylés, et de préférence dans lequel les alcools éthoxylés sont sous forme solide à température ambiante.

13. Papier mousseline selon la revendication 1 dans lequel la composition assouplissante pour papier mousseline comprend de 50 à 70 pour cent en poids d'un amino-polysiloxane et de 30 à 50 pour cent en poids de polyéthylèneglycol, et de préférence dans lequel le polyéthylèneglycol est un solide à température ambiante.

14. Papier mousseline selon la revendication 1 dans lequel la composition assouplissante pour papier mousseline comprend de 50 to 70 pour cent en poids d'un amino-polysiloxane et de 30 à 50 pour cent en poids d'un mélange d'au moins un polyéthylèneglycol et d'au moins un dérivé d'alkyle gras, et de préférence dans lequel le polyéthylèneglycol et le dérivé d'alkyle gras sont sous forme solide à température ambiante.

15. Papier mousseline selon la revendication 1 dans lequel le dérivé d'alkyle gras est le stéarate de glycérol, le dilaurate de glycérol, le monopalmitate de sorbitane, le tristéarate de sorbitane, le sesquioléate de sorbitane, le palmitate de sorbitane polyéthoxylé, l'alcool tridécylique éthoxylé à 9-EO, le céteth-10, le céteth-12 ou le céteth-20.
